# EUROPEAN PATENT SPECIFICATION

(11) **EP 2 682 809 B1**
(45) Date of publication and mention of the grant of the patent: **20.05.2015**
(21) Application number: 12186461.5
(22) Date of filing: 28.09.2012
(51) Int. Cl.: G02C 13/00, A61B 3/11

(54) **Device for measuring parameters for manufacturing spectacle lens**
Vorrichtung zum Messen von Parametern zur Herstellung eines Brillenglases
Dispositif pour mesurer des paramètres de fabrication de lentille de lunettes

(30) Priority: 06.07.2012 KR 20120074085
(43) Date of publication of application: 08.01.2014
(73) Proprietor: ViewITech Co., Ltd., Gwacheon-si Gyeonggi-do (KR)
(72) Inventor: Kweon, Hyuk Je, Gyeonggi-do (KR)
(74) Representative: Ciceri, Fabio

(56) References cited:
- EP-A2- 1 038 495
- WO-A1-99/01791
- WO-A1-2009/086860
- WO-A1-2012/038676

## Description

### CROSS REFERENCE TO RELATED APPLICATION AND CLAIM OF PRIORITY

This application claims the benefit of Korean Patent Application No. 10-2012-0074085, filed on July 6, 2012, in the Korean Intellectual Property Office.

### BACKGROUND OF THE INVENTION

### 1. Field of the Invention

The present invention relates to a device for measuring parameters for manufacturing spectacle lenses.

### 2. Background Art

In order to precisely manufacture spectacle lenses, particularly, special spectacle lenses such as progressive lenses or multi-focal progressive lenses, physical features and habits of wearing glasses of a measurement subject who will wear glasses, such as long-distance or near-distance eye position and direction, a binocular pupil distance, a facial structure, a reading habit, a pupil rotation angle, a face tilt, and so on, must be considered. Such considerations are called 'parameters for manufacturing spectacles", and the parameters for manufacturing spectacles include, for instance, an infinite distance binocular pupil distance, an infinite distance monocular pupil distance, a near distance binocular pupil distance, a near distance monocular pupil distance, a vertical distance and a horizontal distance from the optical center to the pupil center (u/v), a horizontal distance of a lens insertion part of a spectacle frame (BOX A), a vertical distance of the lens insertion part of the spectacle frame (BOX B), the maximum distance from the optical center to the lens insertion part (BOX ED), a horizontal distance between lenses, i.e., right and left lens insertion parts (DBL), a vertical distance from the pupil center to the lower lens insertion part (Eye point), a face tilting angle in a state where the measurement subject stares at the front, a pantoscopic tilt that is formed between a vertical line perpendicular to a lateral reference horizontal plane of the measurement subject and a side line of the spectacle lens, a vertex cornea distance that is a distance from the lateral reference cornea vertex of the measurement subject to the optical center of the spectacle lens, a face form angle that is an angle between an imaginary line from the spectacle frame center to the longest right or left distance of the spectacle lens bent to the right or the left toward the measurement subject's face and an imaginary line horizontally extending from the spectacle frame center to the right or left, a face rotation angle in a state where the measurement subject stares at the front, and so on, but the parameters are not restricted to the above.

Such parameters for manufacturing spectacle lenses must be precisely measured and applied to spectacle lenses when not only general spectacle lenses but also special spectacle lenses, such as progressive lenses or multi-focal progressive lenses, are manufactured. In this context, Korean Patent Laid-open No. 10-2004-0030594 discloses a method of designing spectacle lenses in consideration of eye movements (listing side). In order to easily obtain a high-performance spectacle lens, Korean Patent Laid-open No. 10-2004-0030594 discloses a method of designing spectacle lenses and spectacle lenses manufactured by the same, which uses a vision evaluation function (log MAR) generally drawn from an actually measured vision measurement value V as a vision evaluation function constituting the vertex function used for optimal calculation, wherein the vision evaluation function (log MAR) is indicated by the following formula (1): vision evaluation function (log MAR)= log10(1/V(upper curve, and remaining astigmatism))) when the upper curve is an aberration of a general spectacle lens and the remaining astigmatism is the astigmatism expandably defined by the spectacle lens design considering the listing side. Moreover, Korean Patent Laid-open No. 10-2009-0066296 discloses a device and a method for determining at least one component of directions of corrective spectacle lenses for a person who will wear glasses. In Korean Patent Laid-open No. 10-2009-0066296, the method of optically designing corrective lenses includes a step of installing a position determining system on a frame or a sample lens mounted on the frame, wherein the position determining system includes the steps of: containing at least one discrimination element having at least one geometrical feature of at least one base; two-dimensionally capturing an image of the vertical element on a vertical plane of the face; processing the captured image and measuring geometrical features of the captured image of the discrimination element depending on the geometrical features of the base of the discrimination element; and comparing the captured geometrical features with the geometrical features of the base and calculating at least one component of directions of the lens.

However, because the prior arts adopt complicated mathematical formula or algorithm in order to measure parameters for manufacturing spectacle lenses, a process for manufacturing the device for realizing them is very complicated and a large-sized device is demanded. Furthermore, the prior arts have a problem in that they can measure only one or only a part of parameters for manufacturing spectacle lenses and cannot simultaneously measure various parameters for manufacturing spectacle lenses. Additionally, the prior arts do not consider measurement subjects' unique physical features or spectacle-wearing habits. Accordingly, a method and a device for measuring parameters for manufacturing spectacle lenses which can reflect measurement subjects' unique physical features or spectacle-wearing habits, be more accurately and easily operated, and realize a small-sized portable measuring device.

### SUMMARY

Accordingly, one or more embodiments of the present invention has been made to solve one or more of the above-mentioned problems occurring in the prior art.

In the present invention, there is provided an auxiliary device for measuring parameters for manufacturing spectacle lenses, which is to be installed on a portable computer such as a tablet PC, including: an infinite distance optical module having a target light source and an infinite focus lens arranged in front of the target light source for providing an infinite distance focus to the measurement subject's both eyes from the target light source through the infinite focus lens, and a detachable mount unit by which the infinite distance optical module is to be detachably mounted on the portable tablet computer in such a way that the infinite distance focus providing direction is the same with the capturing direction of the first built-in camera module of the portable computer.

The auxiliary device may further include a first flash module for providing a first flash when the first built-in camera module takes a picture.

The auxiliary device may further include a first built-in flash module for providing a first flash when the first built-in camera module takes a picture.

The auxiliary device may further include an optical path change module for arranging the infinite distance focus providing direction of the infinite focus optical module and the capturing direction of the first built-in camera module in the same vertical area.

The auxiliary device may further include a camera module for taking a picture of the measurement subject who looks at a screen output outputted from a screen module of the portable computer.

The auxiliary device may further include a second built-in flash module for providing a second flash when the camera module takes a picture.

The auxiliary device may further include a second built-in flash module for providing a second flash for providing a second flash when the portable computer has a second built-in camera module and a screen module to output a screen output, and the second built-in camera module takes a picture on premise that the portable tablet computer includes the screen module for outputting the screen output and a second built-in camera module for taking a picture of the measurement subject who looks at the screen output.

A cable terminal of the infinite focus optical module is connected with an audio jack port of the portable computer.

According to the present invention, the auxiliary device for measuring the parameters for manufacturing spectacle lenses can accurately and easily measure various parameters for manufacturing spectacle lenses by being connected with the portable tablet computer.

### BRIEF DESCRIPTION OF THE DRAWINGS

The above and other aspects, features and advantages of the present invention will be apparent from the following detailed description of the embodiments of the invention in conjunction with the accompanying drawings, in which:
FIG. 1 illustrates a near distance focus provided to a measurement subject's both eyes when the measurement subject looks at a target placed at a near distance;
FIG. 2 illustrates an infinite distance focus provided to the measurement subject's both eyes when the measurement subject looks at a target light source placed at the near distance through an infinite focus lens;
FIG. 3 illustrates taking a photograph of the measurement subject's face with a camera module when the measurement subject looks at the infinite distance focus of FIG. 2;
FIG. 4 illustrates the provision of a flash in the photographing of FIG. 3;
FIG. 5 illustrates an indication of light reflection points on the measurement subject's both eyes through the flash in the photographing step of FIG. 4;
FIG. 6 illustrates a reference ruler that the measurement subject wears for measuring the measurement subject's face in FIGS. 2 to 5;
FIG. 7 illustrates a photograph obtained when the measurement subject looks at an infinite distance focus after wearing the reference ruler of FIG. 6;
FIGS. 8 to 19 illustrate parameters for manufacturing spectacle lenses that are confirmed through the method for measuring parameters according to an example not covered by the invention;
FIGS. 20 to 24 are schematic views showing a device for measuring parameters for manufacturing spectacle lenses according to an example not covered by the invention;
FIGS. 25 and 26 illustrate an optical path change module according to an example not covered by the invention; and
FIGS. 27 to 29 illustrate an auxiliary device for measuring parameters for manufacturing spectacle lenses that is installed on a portable tablet computer to measure parameters for manufacturing spectacle lenses.

### DETAILED DESCRIPTION

Reference will be now made in detail to the embodiment of the present invention with reference to the attached drawings. It would be understood that the following description is provided just to make those skilled in the art easily understand the particular embodiment of the present invention and does not restrict the scope of claims of the present invention.

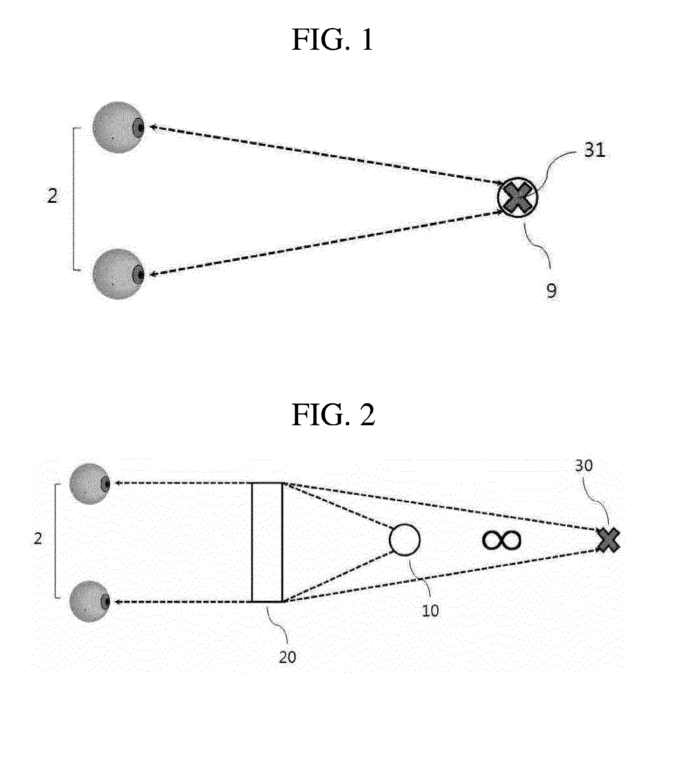

FIG. 1 illustrates a near distance focus 31 provided to a measurement subject's both eyes 2 when the measurement subject looks at a target 9 placed at a near distance. In FIG. 1, when the measurement subject looks at the near distance target 9, namely, the target 9 that is arranged at the near distance, the measurement subject's both eyes 2, namely, a binocular pupil distance, are collected toward the near distance focus 31 to thereby generate a "pupil deflection". The pupil deflection is a phenomenon generated in the case that a person looks at a thing arranged at a distance of about 1m to 5m or that the person reads a book or looks at a thing in his or her hand, but is not generated when the person ordinarily looks at a thing arranged at a long distance or does not look at anything in particular. Accordingly, in order to manufacture spectacle lenses, the most primary consideration is to grasp a lens area for binocular vision in a state where the measurement subject looks at a long distance or an infinite distance without the pupil deflection phenomenon. However, in order to provide the measurement subject with the long distance or infinite distance focus, there is a limit in a measuring place and a large-sized device must be used because a target must be arranged considerably far away from the measurement subject. Moreover, in the case that the measurement subject uses the target arranged at a short distance, as described above, it is impossible to accurately measure because the pupil deflection phenomenon is generated in the measurement subject's both eyes. In order to solve the above problems, the present invention provides an infinite distance focus to show the same effect as the case that the measurement subject looks at a target arranged at a long distance or at an infinite distance. In the meantime, in this specification, the "short distance" (which is also referred to as "near distance") means a distance within a range of about 1 meter, for example, about 50 centimeter from the measurement subject and the "long distance" or the "infinite distance" means a distance which is farther than the short distance, namely, over about 5 meter from the measurement subject.

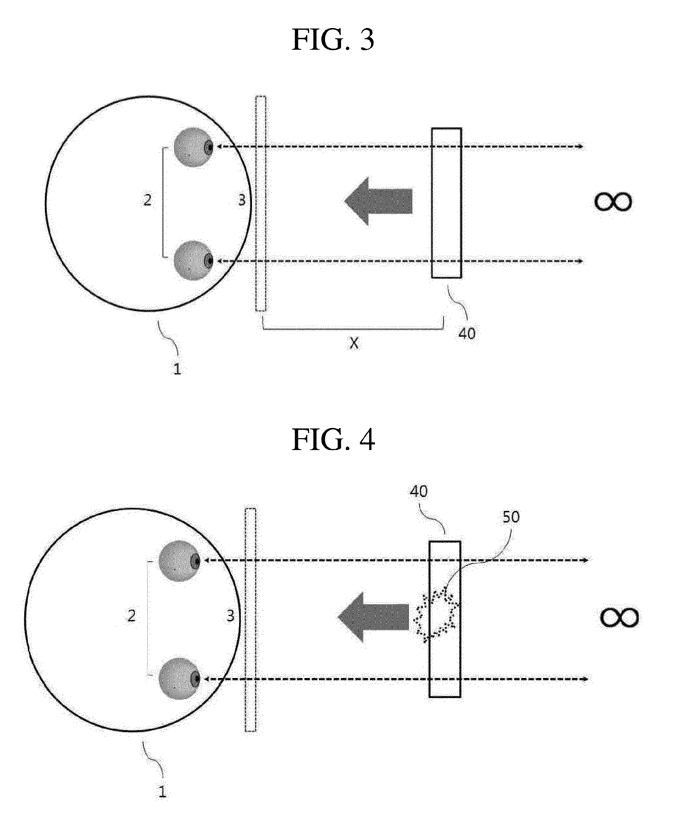

FIG. 2 illustrates an infinite distance focus 30 provided to the measurement subject's both eyes 2 when the measurement subject looks at a target light source 10 placed at the near distance through an infinite focus lens 20, and FIG. 3 illustrates taking a photograph of the measurement subject's face 3 with a camera module 40 when the measurement subject 1 looks at the infinite distance focus 30 of FIG. 2.

A method for measuring parameters for manufacturing spectacle lenses according to an example not covered by the invention includes: providing an infinite distance focus 30 to the measurement subject's both eyes 2 from a target light source 10 through an infinite focus lens 20; taking a picture of the measurement subject's face 3 with a camera module 40 when the measurement subject 1 looks at the infinite distance focus 30; and measuring the parameters for manufacturing spectacle lenses for the measurement subject on the basis of an image of the measurement subject's face 3.

According to FIG. 2, the method for measuring parameters for manufacturing spectacle lenses includes providing the infinite distance focus 30 to the measurement subject's both eyes 2 from the target light source 10 through the infinite focus lens 20. The target light source 10 may be one of various means or modules for providing light, for instance, light-emitting diode (LED) elements, in detail, flash LED elements, infrared ray elements, and so on. The infinite focus lens 20 may be a concave lens, a convex lens or a combined lens where the concave lens and the convex lens are combined, or may be realized in various ways within a range of the technical scope of the present example utilizing optical path changing means or modules which can provide the infinite distance focus to the measurement subject's both eyes 2. The target light source 10 may be arranged at an arbitrary position, and the infinite focus lens 20 may be arranged at a predetermined distance from the target light source 1 out of directions toward the measurement subject's both eyes 2. When light passing through the infinite focus lens 20 from the target light source 10 is provided to the measurement subject, the infinite distance focus 30 is made on the measurement subject's both eyes 2. That is, the target light source 10 arranged at the short distance is seen as a thing arranged at the infinite distance in the measurement subject's both eyes. As a result, even though the target light source 10 is arranged at the short distance, in fact, because the measurement subject who looks at the target light source 10 becomes a state that he or she looks at the target light source 10 at the infinite distance, the pupil distortion is not generated to the measurement subject's both eyes 2 and the measurement subject keeps a normal pupil condition of the eyes 2.

According to FIG. 3, the method for measuring parameters for manufacturing spectacle lenses includes taking a picture of the measurement subject's face 3 with the camera module 40 when the measurement subject 1 looks at the infinite distance focus 30. The camera module 40 includes a module which can take a picture of the face 3 of the measurement subject 1 who looks at the infinite distance focus 30. The camera module 40 may include a module which can convert the image captured by a camera into digital information and easily store, process or analyze the digital information. Because the camera module 40 directly captures the face 3 of the measurement subject 1 who looks at the infinite distance focus 30, an inspector can easily grasp the measurement subject's physical features such as the face structure and spectacle-wearing habits of the measurement subject who looks at the infinite distance focus 30. Meanwhile, a distance (X) from the face 3 of the measurement subject 1 who looks at the infinite distance focus 30 to the camera module 40 can be remarkably reduced, for instance, below 0.5 meter, namely, 50 centimeter. Accordingly, the measurement subject 1 and the inspector (not shown in drawings) can measure parameters for manufacturing spectacle lenses even in a narrow space.

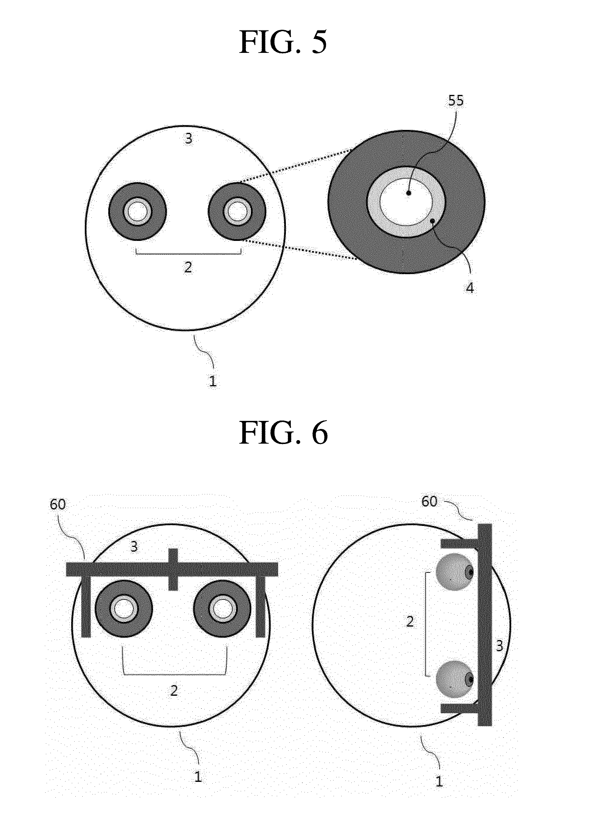

In the meantime, according to FIG. 4, the method for measuring parameters may further include providing the flash 50 by a flash module while taking a picture of the measurement subject's face 3 and indicating light reflection points on the pupils of the measurement subject's both eyes 2. Of course, the flash module (not shown) may be built in the camera module 40 or externally mounted on the camera module 40. As described above, when the camera takes the picture of the measurement subject's face 3, the flash 50 is provided so as to indicate the light reflection points on the pupils of the measurement subject's both eyes 2, so that the inspector can easily grasp physical information on the measurement subject's face and it makes the degree of precision higher in measuring the parameter for manufacturing spectacle lenses. In other words, referring to FIG. 5, if the image shown in FIG. 5 is assumed to be a captured image of a face 3 of the measurement subject 1, the light reflection points 55 formed by the flash 50 are indicated on the pupils 4 of the measurement subject's eyes 2. The light reflection points 55 may become reference points for measuring the measurement subject's infinite distance binocular pupil distance, the measurement subject's infinite distance monocular pupil distance, a distance from the measurement subject's both eyes 2 to the camera module 40, and so on.

Referring to FIG. 6, photography can be made in a state where the measurement subject 1 wears a reference ruler 60 for face measurement of the measurement subject 1. The face measurement means to measure and calculate physical features of the measurement subject's face. The reference ruler 60 is captured together with the measurement subject's face 3 and provides a horizontal reference line and a vertical reference line to measure and calculate the physical features of the measurement subject's face 3 and serves to provide actual measurement units, such as the face length, face height, and so on.

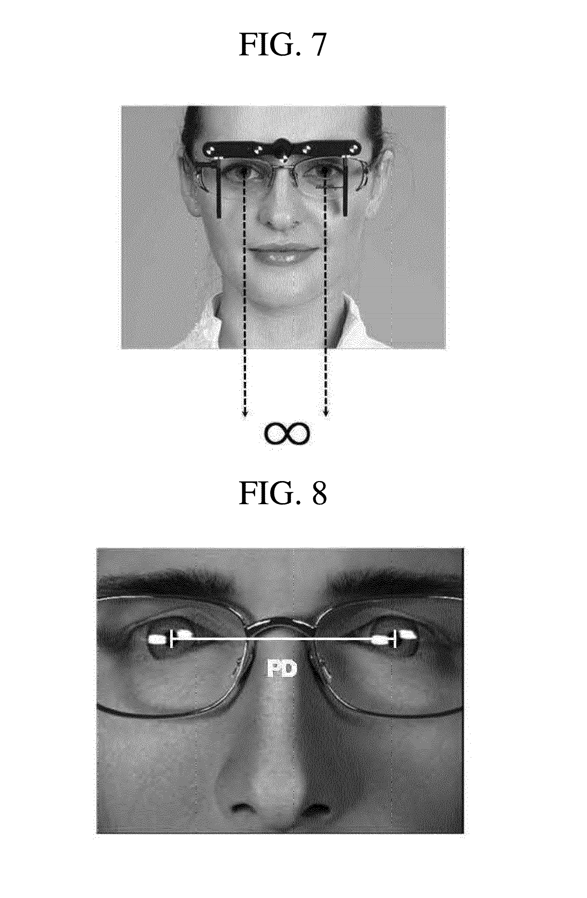

FIG. 7 illustrates an actual photograph of the face obtained when the measurement subject looks at the infinite distance focus 30 after wearing the reference ruler 60. In FIG. 7, because the measurement subject 1 looks at the infinite distance focus 30, the measurement subject keeps the binocular pupil distance in the normal condition without pupil distortion, and in the above state, the physical features of the measurement subject's face can be easily measured and grasped by the reference ruler 60. Meanwhile, referring to FIG. 7, the measurement subject 1 wears spectacles, but, even in the state where the measurement subject 1 does not wear the spectacles, photography is possible and the parameters for manufacturing spectacle lenses can be measured.

The method for measuring parameters for manufacturing spectacle lenses according to present example includes measuring the parameters for manufacturing spectacle lenses on the basis of the captured image of the measurement subject's face 3.

FIGS. 8 to 19 illustrate parameters for manufacturing spectacle lenses that are confirmed through the method for measuring parameters according to the present example.

FIG. 8 illustrates the infinite distance binocular pupil distance of the measurement subject, who looks at the infinite distance focus, which is measurable through the captured image, namely, an infinite distance binocular pupil distance (PD). Not shown in FIG. 8, but an infinite distance monocular pupil distance of the measurement subject who looks at the infinite distance focus, namely, an infinite distance monocular pupil distance, can be measured by calculating a value of the infinite distance binocular pupil distance in half or by actually measuring the captured image.

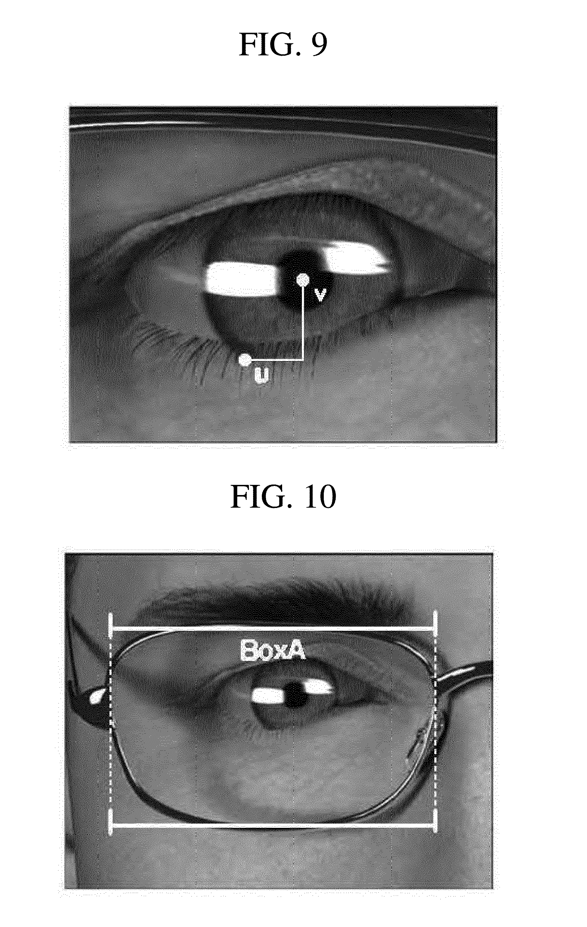

FIG. 9 illustrates a vertical distance and a horizontal distance (u/v) being measurable through the captured image and ranging from the center point of a spectacle frame or the center point of the spectacle lens of the measurement subject, who looks at the infinite distance focus, to the center of the pupil or the reflection point of the eyeball. In this instance, the captured image may be an actual image that directly took a picture of the measurement subject who wears spectacles or may be made by overlapping virtual spectacle images after taking a picture of the measurement subject who does not wear spectacles.

FIG. 10 illustrates a horizontal eye size (BOX A) of a lens insertion part of the spectacle frame of the measurement subject, who looks at the infinite distance focus, which is measurable through the captured image. In this instance, the captured image may be an actual image that directly took a picture of the measurement subject who wears spectacles or may be made by overlapping virtual spectacle images after taking a picture of the measurement subject who does not wear spectacles.

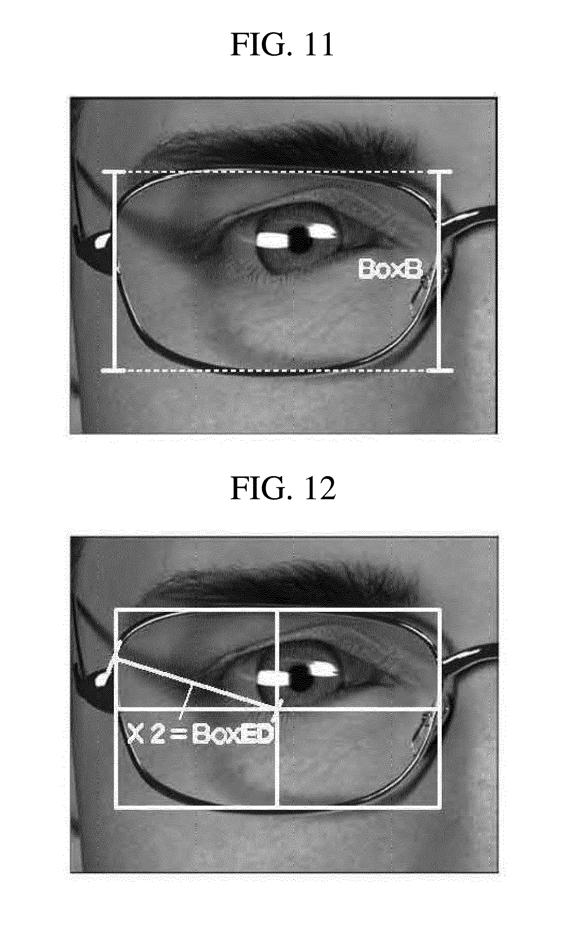

FIG. 11 illustrates a vertical eye size (BOX B) of a lens insertion part of the spectacle frame of the measurement subject, who looks at the infinite distance focus, which is measurable through the captured image. In this instance, the captured image may be an actual image that directly took a picture of the measurement subject who wears spectacles or may be made by overlapping virtual spectacle images after taking a picture of the measurement subject who does not wear spectacles.

FIG. 12 illustrates the maximum diameter of the lens (BOX ED) being measurable through the captured image and ranging from the center point of the spectacle frame to the lens insertion part of the measurement subject who looks at the infinite distance focus. In this instance, the captured image may be an actual image that directly took a picture of the measurement subject who wears spectacles or may be made by overlapping virtual spectacle images after taking a picture of the measurement subject who does not wear spectacles.

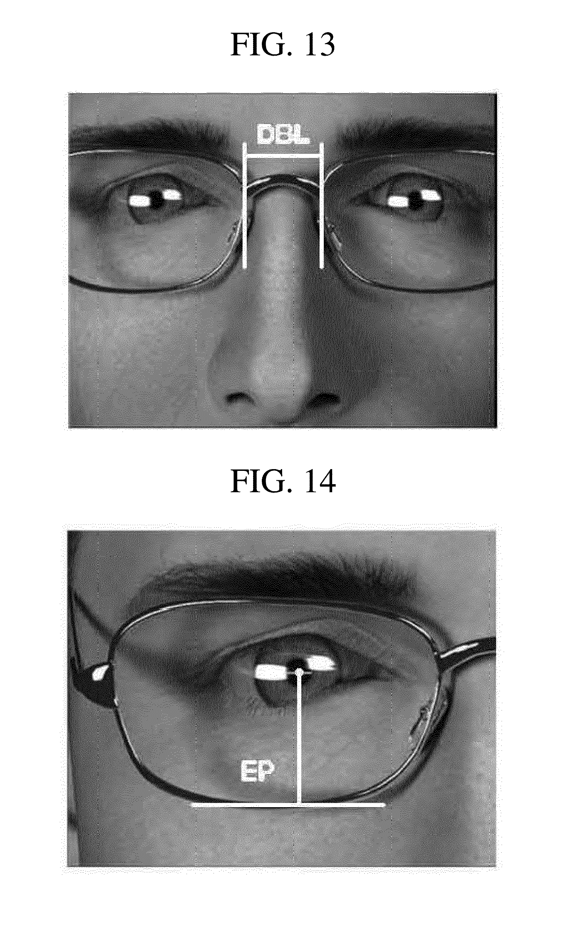

FIG. 13 illustrates a horizontal distance between lenses (DBL) being measurable through the captured image and ranging between right and left lens insertion parts of the measurement subject who looks at the infinite distance focus. In this instance, the captured image may be an actual image that directly took a picture of the measurement subject who wears spectacles or may be made by overlapping virtual spectacle images after taking a picture of the measurement subject who does not wear spectacles.

FIG. 14 illustrates an eye point (EP) being measurable through the captured image and ranging from the pupil center of the measurement subject, who looks at the infinite distance focus, to a lower end lens insertion part. In this instance, the captured image may be an actual image that directly took a picture of the measurement subject who wears spectacles or may be made by overlapping virtual spectacle images after taking a picture of the measurement subject who does not wear spectacles.

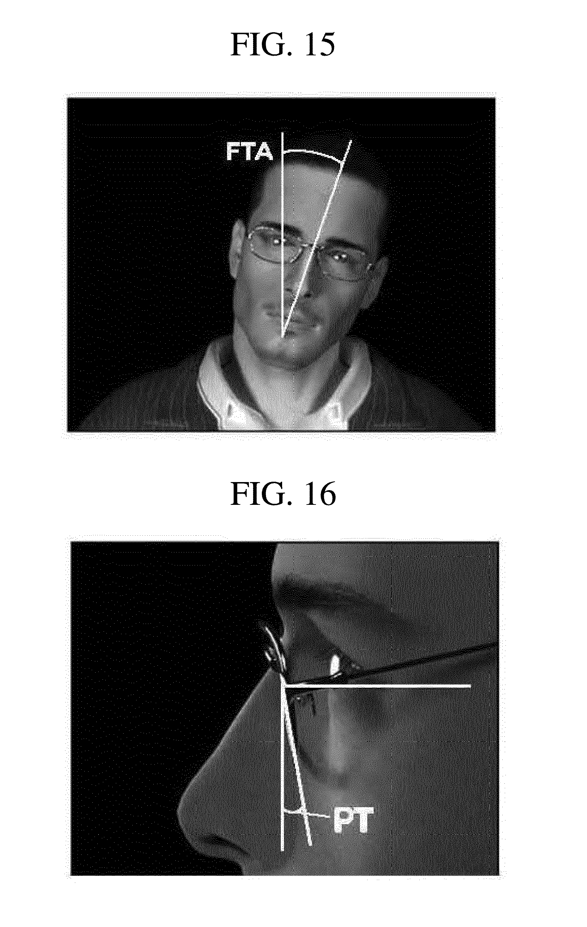

FIG. 15 illustrates a face tilting angle (FTA) being measurable through the captured image in a state where the measurement subject who looks at the infinite distance focus stares at the front. In this instance, the captured image may be an actual image that directly took a picture of the measurement subject who wears spectacles or may be made by overlapping virtual spectacle images after taking a picture of the measurement subject who does not wear spectacles.

FIG. 16 illustrates a pantoscopic tilt (PT) being measurable through the captured image of the side of the measurement subject who looks at the infinite distance focus and being formed between a vertical line perpendicular to a lateral reference horizontal plane of the measurement subject and a side line of the spectacle lens. In this instance, the captured image may be an actual image that directly took a picture of the measurement subject who wears spectacles or may be made by overlapping virtual spectacle images after taking a picture of the measurement subject who does not wear spectacles.

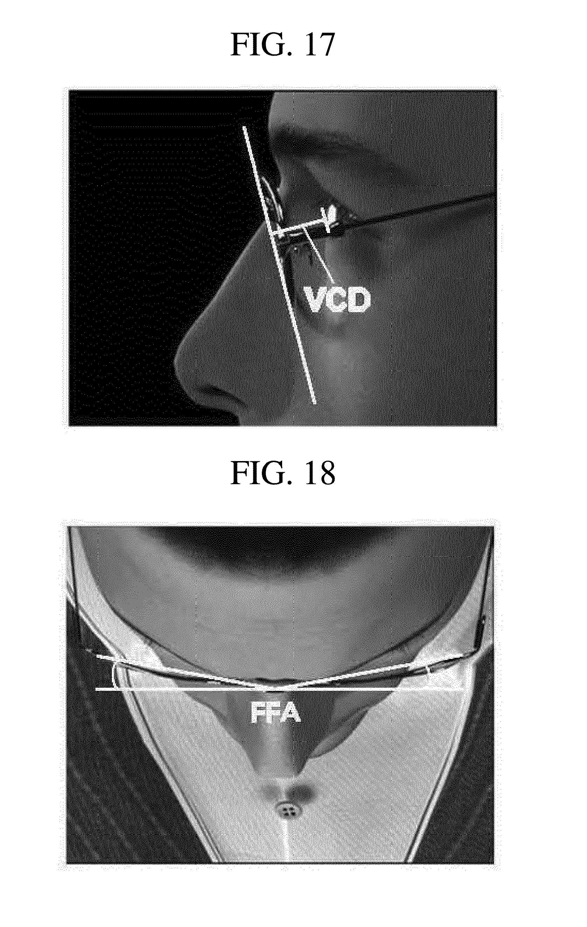

FIG. 17 illustrates a vertex cornea distance (VCD) being measurable through the captured image of the side of the measurement subject who looks at the infinite distance focus and ranging from the measurement subject's lateral reference cornea vertex to the optical center of the spectacle lens. In this instance, the captured image may be an actual image that directly took a picture of the measurement subject who wears spectacles or may be made by overlapping virtual spectacle images after taking a picture of the measurement subject who does not wear spectacles.

FIG. 18 illustrates a face form angle (FFA) being measurable through the captured image of the top of the measurement subject who looks at the infinite distance focus and being formed between an imaginary line, which ranges from the spectacle frame center to the longest right or left distance of the spectacle lens bent to the right or the left toward the measurement subject's face, and an imaginary line horizontally extending from the spectacle frame center to the right or left when the measurement subject wears the spectacles. In this instance, the captured image may be an actual image that directly took a picture of the measurement subject who wears spectacles or may be made by overlapping virtual spectacle images after taking a picture of the measurement subject who does not wear spectacles.

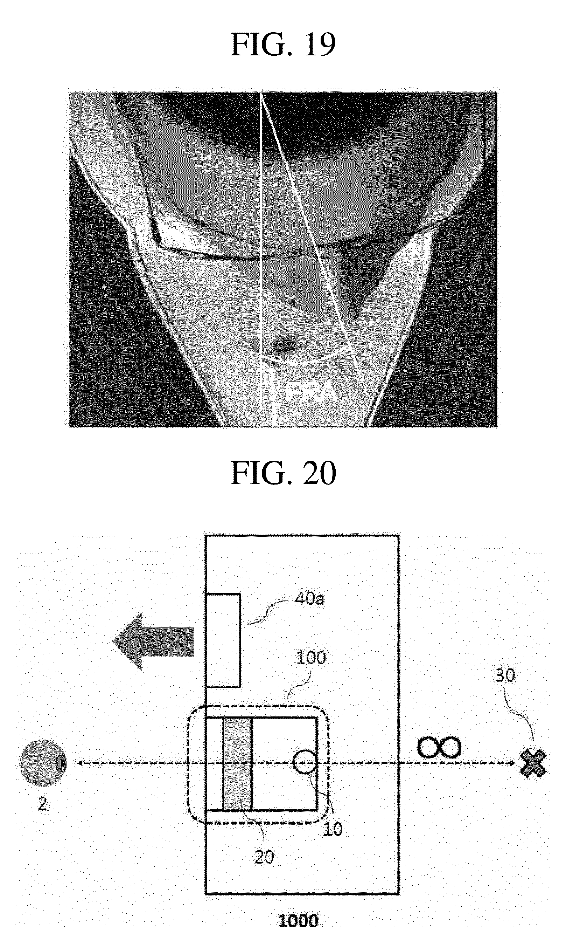

FIG. 19 illustrates a face rotation angle (FRA) being measurable through the captured image of the top of the measurement subject who looks at the infinite distance focus in a state where the measurement subject stares at the front. In this instance, the captured image may be an actual image that directly took a picture of the measurement subject who wears spectacles or may be made by overlapping virtual spectacle images after taking a picture of the measurement subject who does not wear spectacles.

FIGS. 20 to 24 are schematic views showing a device for measuring parameters for manufacturing spectacle lenses according to an example not covered by the invention.

The device 1000 for measuring parameters for manufacturing spectacle lenses based on the captured image of the measurement subject according to the example includes: an infinite distance optical module 100 having a target light source 10 and an infinite focus lens 20 arranged in front of the target light source 10 for providing an infinite distance focus 30 to the measurement subject's both eyes 2 from the target light source 10 through the infinite focus lens 20; and a first camera module 40a for making a picture of the measurement subject 1 who looks at the infinite focus lens 20.

Referring to FIG. 20, the infinite distance optical module 100 may include the target light source 10, the infinite focus lens 20, and a housing module for accommodating them. The target light source 10 may be one of various means or modules for providing light, for instance, light-emitting diode (LED) elements, in detail, flash LED elements, infrared ray elements, and so on. The infinite focus lens 20 may be a concave lens, a convex lens or a combined lens where the concave lens and the convex lens are combined, or may be realized in various ways within a range of the technical scope of the present example utilizing optical path changing means or modules which can provide the infinite distance focus to the measurement subject's both eyes 2. The first camera module 40a is to take a picture of the measurement subject who looks at the infinite distance focus 20, for example, a camera module of a type which can convert the image captured by a camera into digital information and easily store, process or analyze the digital information. Referring to FIG. 20, the device 1000 for measuring parameters for manufacturing spectacle lenses based on the captured image of the measurement subject according to the example is arranged in front of the measurement subject's both eyes 2, and light is provided from the target light source 10 in such a way as to pass the infinite focus lens 20, and then, the measurement subject looks at the infinite distance focus 30 without any pupil distortion. After that, in this condition, when the first camera module 40a takes a picture of the measurement subject who looks at the infinite distance focus 30, an image of the measurement subject's face can be obtained, and the device can measure the parameters for manufacturing spectacle lenses of the measurement subject on the basis of the obtained image.

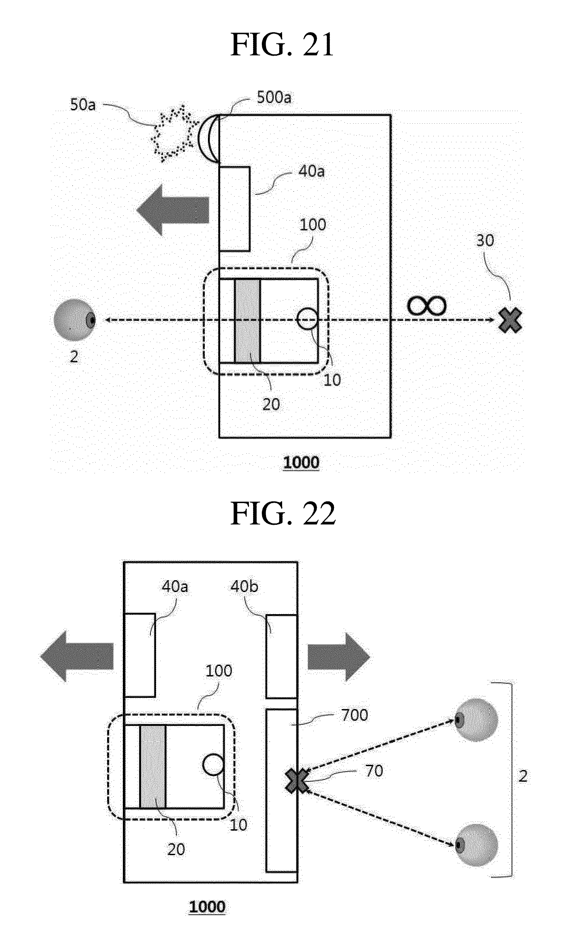

Referring to FIG. 21, the device 1000 for measuring parameters for manufacturing spectacle lenses based on the captured image of the measurement subject according to the example further includes a first flash module 500a for providing a first flash 50a when the first cameral module 40a takes a picture. The first flash module 500a may be built in the first camera module 40a or externally mounted on the first camera module 40a. When the camera takes the picture of the measurement subject's face 3, the first flash 50a is provided to indicate the light reflection points on the pupils of the measurement subject's both eyes 2, so that the inspector can easily grasp physical information on the measurement subject's face and it makes the degree of precision higher in measuring the parameter for manufacturing spectacle lenses. The light reflection points may become reference points for measuring the measurement subject's infinite distance binocular pupil distance, the measurement subject's infinite distance monocular pupil distance, a distance from the measurement subject's both eyes 2 to the camera module 40, and so on. In the meantime, the first flash module 50a may be one of various means or modules, for example, light-emitting diode (LED) elements, in detail, flash LED elements, infrared ray (IR) elements, and so on.

Referring to FIG. 22, the device 1000 for measuring parameters for manufacturing spectacle lenses based on the captured image of the measurement subject according to the example may further include: a screen module 700 for providing a near distance focus 70 to the measurement subject's both eyes 2 through a screen output; and a second camera module 40b for taking a picture of the measurement subject who looks at the near distance focus 70. The screen module 700 outputs images on a certain screen, serves to provide the near distance focus to the measurement subject, and may be realized in various ways. The screen module 700, for instance, can output e-book contents (screen output). The near distance focus means a point where focuses of the measurement subject's binocular pupils in a state where the measurement subject looks at a thing arranged at the near distance. Accordingly, the screen module 700 outputs the screen output and provides the near distance focus 70 to the measurement subject's both eyes 2 in a state where the measurement subject looks at the screen output in a comfortable position. Moreover, the second camera module 40b may be a camera module of a type which can convert the image captured by a camera into digital information and easily store, process or analyze the digital information. Referring to FIG. 22, the device 1000 for measuring parameters for manufacturing spectacle lenses based on the captured image of the measurement subject according to the example is arranged in front of the measurement subject's both eyes 2, and the measurement subject looks at the near distance focus 70 while looking at the screen output when the screen module 700 provides the screen output. After that, in this condition, when the second camera module 40b takes a picture of the measurement subject who looks at the near distance focus 70, an image of the measurement subject's face can be obtained, and on the basis of the obtained image, the device can measure the parameters for manufacturing spectacle lenses of the measurement subject, for instance, the near distance binocular pupil distance, the near distance monocular pupil distance, and so on.

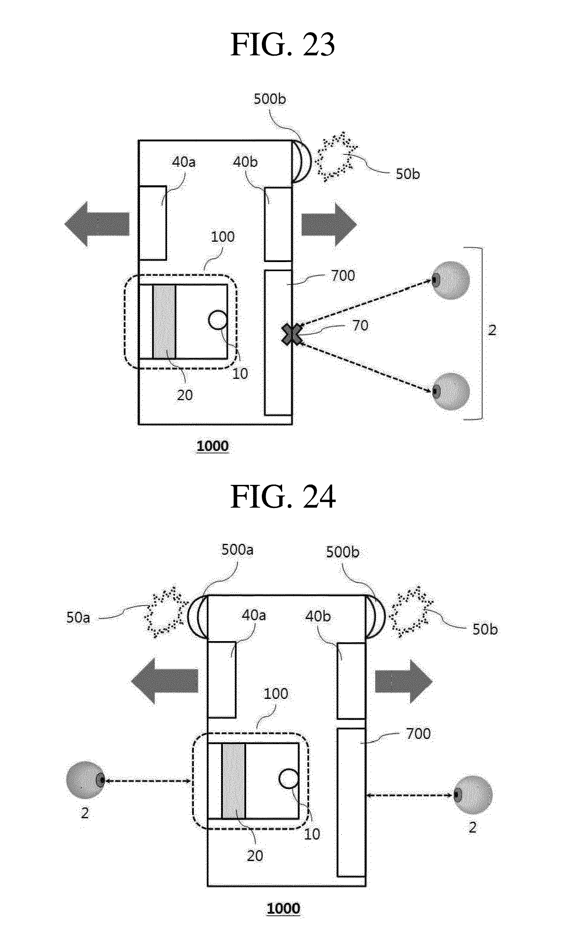

Referring to FIG. 23, the device 1000 for measuring parameters for manufacturing spectacle lenses based on the captured image of the measurement subject according to the further further includes a second flash module 500b for providing a second flash 50b when the second cameral module 40b takes a picture. The second flash module 500b may be built in the second camera module 40b or externally mounted on the second camera module 40b. When the camera takes the picture of the measurement subject's face 3, the second flash 50b is provided to indicate the light reflection points on the pupils of the measurement subject's both eyes 2, so that the inspector can easily grasp physical information on the measurement subject's face in the state where the measurement subject looks at the near distance focus 70 and it makes the degree of precision higher in measuring the parameter for manufacturing spectacle lenses. The light reflection points may become reference points for measuring the measurement subject's infinite distance binocular pupil distance, the measurement subject's infinite distance monocular pupil distance, a distance from the measurement subject's both eyes 2 to the camera module 40, and so on. In the meantime, the second flash module 50b may be one of various means or modules, for example, light-emitting diode (LED) elements, in detail, flash LED elements, infrared ray elements, and so on.

Referring to FIG. 24, the device 1000 for measuring parameters for manufacturing spectacle lenses based on the captured image of the measurement subject according to the example includes: an infinite distance optical module 100 having a target light source 10 and an infinite focus lens 20 arranged in front of the target light source 10 for providing an infinite distance focus 30 to the measurement subject's both eyes 2 from the target light source 10 through the infinite focus lens 20; a first camera module 40a for making a picture of the measurement subject 1 who looks at the infinite focus lens 20; a first flash module 500a for providing a first flash 50a when the first camera module 40a takes a picture; a screen module 700 for providing a near distance focus 70 to the measurement subject's both eyes 2 through the screen output; a second camera module 40b for taking a picture of the measurement subject who looks at the near distance focus 70; and a second flash module 500b for providing a second flash 50b when the second camera module 40b takes a picture. Furthermore, not shown in FIGS. 20 to 24, but the device 1000 for measuring parameters for manufacturing spectacle lenses based on the captured image of the measurement subject according to the embodiment of example may further include a reference ruler module for measuring the measurement subject's face in a state where the measurement subject wears the reference ruler module. The reference ruler module is previously described referring to FIG. 6.

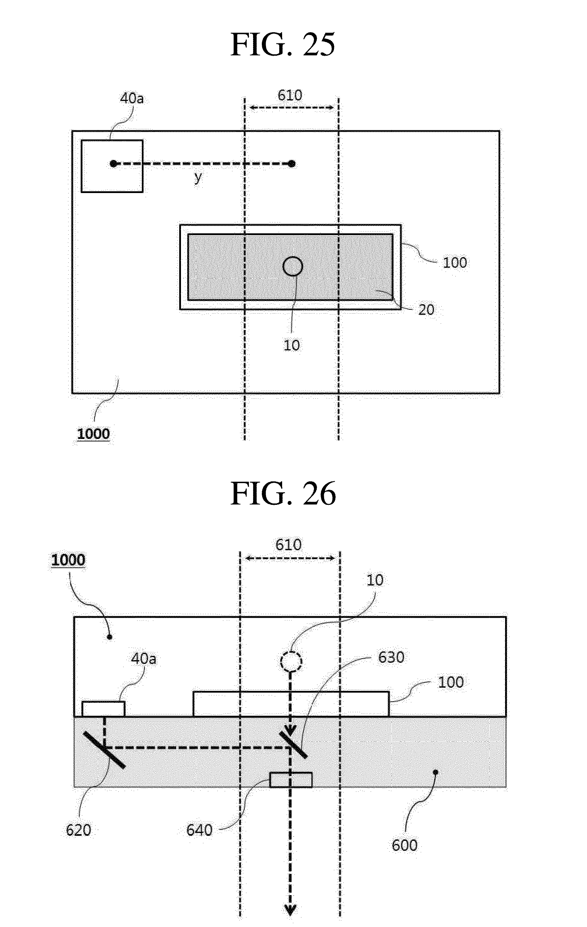

FIGS. 25 and 26 illustrate an optical path change module according to the example.

FIG. 25 is a front view of the device 1000 for measuring parameters for manufacturing spectacle lenses based on the captured image of the measurement subject according to the present example. Referring to FIG. 25, because a capturing direction (front direction) of the first camera module 40a of the measuring device 1000 and an infinite distance focus providing direction (front direction) of the target light source 10 arranged inside the infinite distance optical module 100 and on the rear surface of the infinite focus lens 20 are not arranged in the same vertical area 610, there may be a minute error (y) on the captured image as much as a horizontal distance between the first camera module 40a and the target light source 10 when the first camera module 40a takes a picture. Accordingly, the device 1000 for measuring parameters for manufacturing spectacle lenses based on the captured image of the subject according to the example may further include an optical path change module 600 for arranging the infinite distance focus providing direction of the infinite focus optical module 100 and the capturing direction of the first camera module 40a in the same vertical area 610. FIG. 26 is a top view showing a state where the optical path change module 600 is mounted on the front surface of the device 1000 for measuring parameters for manufacturing spectacle lenses. Referring to FIG. 26, the optical path module 600 includes: a first reflection mirror 620 for primarily contributing to an optical path vertical change from the capturing direction of the first camera module 40a; a second reflection mirror 630 for secondarily contributing to the optical path vertical change from the first reflection mirror 620; and an optical exposure part 640 for exposing light to the front of the vertical area 610 which is the same as the infinite distance focus providing direction of the infinite focus optical module 100 from the second reflection mirror 630. As a result, the optical path module 600 can remove the minute error on the captured image due to a difference in horizontal position between the first cameral module 40a and the target light source 10 when the first camera module 40a takes a picture.

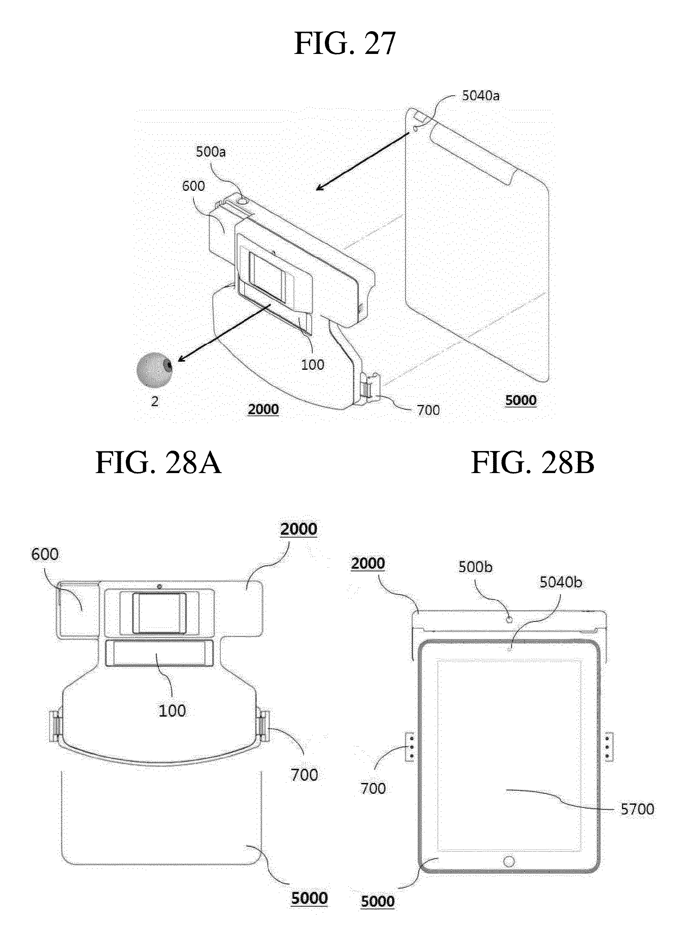

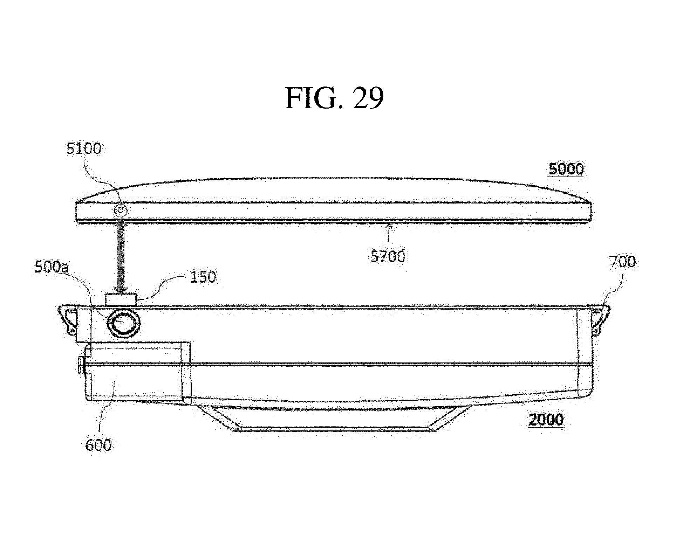

FIGS. 27 to 29 illustrate an auxiliary device 2000 for measuring parameters for manufacturing spectacle lenses that is installed on a portable computer such as a portable tablet computer 5000 to measure parameters for manufacturing spectacle lenses. In detail, FIG. 27 illustrates a state where the auxiliary device 2000 for measuring parameters for manufacturing spectacle lenses is installed on the rear surface of the portable tablet computer 5000, FIGS. 28A and 28B are each a front left side view and a rear right side view showing the state where the auxiliary device 2000 for measuring parameters for manufacturing spectacle lenses is installed on the portable tablet computer 5000, and FIG. 29 illustrates a state where a cable terminal 150 of an infinite focus optical module 100 of the auxiliary device 2000 is connected with an audio jack port 5100 of the portable tablet computer 5000.

The auxiliary device 2000 for measuring parameters for manufacturing spectacle lenses can be installed on the portable tablet computer 5000 in order to measure the parameter for manufacturing spectacle lenses. The portable tablet computer 5000 is a portable computer based on a touch screen, such as iOS-based iPads, Android OS-based Galaxy Tabs, Windows-based tablet computers, and so on. In this embodiment, the portable tablet computer 5000 may include all or some of a screen module having input and output functions, a camera module for taking a picture of surrounding environment in real time, a flash module for providing a flash during photography, a motion sensor for sensing motion patterns by a user's manipulation, and an arithmetic operation module for storing and/or processing information inputted from the screen module, the camera module, the flash module and the motion sensor module. For instance, the screen module has the input and output functions by the user's manipulation and may be arranged on the front face of the portable table computer 5000, and the camera module can capture surrounding environment in real time and be arranged on the front face, the rear face or a part of the front face and the rear face of the portable tablet computer 5000. The flash module may be arranged together with or near to the camera module. The motion sensor module is a module to sense motion patterns by the user's manipulation when the user carries out some motions in a state where the user carries the portable tablet computer 5000, for instance, is an acceleration sensor. The acceleration sensor senses the user's up and down and right and left motions, namely, the portable tablet computer's up and down and right and left motions by the user's motions, to thereby sense parameters, such as a distance, a tilt angle between the user and the portable tablet computer, and so on. The arithmetic operation module can store and process information inputted from the screen module, the camera module, the flash module and the motion sensor module and also store and process information stored in the portable tablet computer or information received through a near distance or long distance network.

Referring to FIG. 27, the auxiliary device 2000 for measuring parameters for manufacturing spectacle lenses according to the present invention is installed on the portable table computer 5000 and includes: an infinite distance optical module 100 having a target light source 10 and an infinite focus lens 20 arranged in front of the target light source 10 for providing an infinite distance focus 30 to the measurement subject's both eyes 2 from the target light source 10 through the infinite focus lens 20, wherein the infinite distance optical module 100 is mounted on the portable tablet computer 5000 through a detachable mounting unit 700 in such a fashion that the infinite distance focus providing direction (an arrow direction at the bottom of the left side) is the same with the capturing direction (an arrow direction at the top of the center) of the first built-in camera module 5040a of the portable tablet computer 5000. In this instance, the infinite distance optical module 100 is the same as the above. The auxiliary device 2000 for measuring parameters for manufacturing spectacle lenses according to an embodiment of the present invention is mounted on the portable tablet computer 5000 through detachable mounting unit 700, and in FIG. 27, the detachable mounting unit 700 fixes the portable tablet computer 5000 while surrounding a partial area of the both side frames of the portable tablet computer 5000, but is not restricted to the above.

The portable tablet computer 5000 may further include a first built-in flash module for providing a first flash when the first built-in camera module 5040a takes a picture. If the portable tablet computer 5000 does not include the first built-in flash module, the auxiliary device 200 for measuring the parameters for manufacturing spectacle lenses according to an embodiment of the present invention may further include a first flash module 500a for providing the first flash when the first built-in camera module 5040a.

The auxiliary device 200 for measuring the parameters for manufacturing spectacle lenses according to an embodiment of the present invention may further include an optical path change module 600 for arranging the infinite distance focus providing direction of the infinite focus optical module 100 and the capturing direction of the first built-in camera module 5040a in the same vertical area. The optical path change module 600 is the same as the above.

Additionally, the auxiliary device 200 for measuring the parameters for manufacturing spectacle lenses according to an embodiment of the present invention may further include a camera module for taking a picture of the measurement subject who looks at a screen module 5700 on the premise that the portable tablet computer 5000 includes the screen module 5700 for outputting a screen output. In this instance, the auxiliary device 200 for measuring the parameters for manufacturing spectacle lenses according to an embodiment of the present invention may further include a second flash module for providing a second flash when the camera module takes a picture. Referring to FIGS. 28A and 28B, the auxiliary device 200 for measuring the parameters for manufacturing spectacle lenses according to an embodiment of the present invention may further include a second built-in flash module 500b for providing a second flash 50b when a second built-in camera module 5040b takes a picture on premise that the portable tablet computer 5000 includes the screen module 5700 for outputting the screen output and the second built-in camera module 5040b for taking a picture of the measurement subject who looks at the screen module 5700.

A cable terminal 150 of the auxiliary device 200 for measuring the parameters for manufacturing spectacle lenses according to an embodiment of the present invention, for instance, the cable terminal 150 of the infinite focus optical module 100 may be connected with an audio jack port 5100 of the portable tablet computer 5000 (See an arrow in the drawing). In this instance, the portable tablet computer 5000 can control operations of the infinite focus optical module 1000 and the optical path change module 600 and/or the second flash module 500b and provide electricity to the auxiliary device 200 for measuring the parameters for manufacturing spectacle lenses according to the present invention.

As described above, one of ordinary skill in the art may understand that the present invention may be embodied in other detailed forms without any change in the technical concept or necessary features thereof. Accordingly, the above-described embodiments are exemplary only on all of the aspects, and are not restricted. The scope of the present invention is defined by the following claims, rather than the detailed description section, and any change or changed forms, originated from the meaning, range, and equivalent concept of the claims, must be interpreted as being included in the scope of the present invention.

## Claims

1. An auxiliary device for measuring parameters for manufacturing spectacle lenses, which is to be installed on a portable computer having a first built-in camera module, the auxiliary device comprising:
an infinite distance optical module comprising a target light source and an infinite focus lens arranged in front of the target light source for providing an infinite distance focus to both eyes of a measurement subject from the target light source through the infinite focus lens; and
- said infinite distance optical module is to be detachably mounted on the portable computer in such a way that the infinite distance focus providing direction is the same as the capturing direction of the first built-in camera module of the portable computer.

2. The auxiliary device according to claim 1, further comprising:
a first flash module for providing a first flash when the first built-in camera module takes a picture.

3. The auxiliary device according to claim 1, further comprising:
a first built-in flash module for providing a first flash when the first built-in camera module takes a picture.

4. The auxiliary device according to claim 1, further comprising:
an optical path change module for arranging the infinite distance focus providing direction of the infinite focus optical module and the capturing direction of the first built-in camera module in the same vertical area.

5. The auxiliary device according to claim 1, further comprising:
a camera module for taking a picture of the measurement subject who looks at a screen output outputted from a screen module of the portable computer.

6. The auxiliary device according to claim 5, further comprising:
a second built-in flash module for providing a second flash when the camera module takes a picture.

7. The auxiliary device according to claim 1, wherein a cable terminal of the infinite focus optical module is connected with an audio jack port of the portable computer.

## Patentansprüche

1. Hilfsvorrichtung zum Messen von Parametern für die Herstellung von Brillengläsern, die auf einem tragbaren Computer zu installieren ist, der ein erstes integriertes Kameramodul aufweist, wobei die Hilfsvorrichtung Folgendes umfasst:
- ein optisches Fernsichtmodul, das eine Ziellichtquelle und eine Linse mit unendlichem Brennpunkt umfasst, die vor der Ziellichtquelle angeordnet ist, um für die beiden Augen einer Messperson von der Ziellichtquelle durch die Linse mit unendlichem Brennpunkt hindurch einen Brennpunkt in unendlicher Entfernung bereitzustellen; und
- wobei das optische Fernsichtmodul derart abnehmbar an dem tragbaren Computer montiert ist, dass die Richtung, die den Brennpunkt in unendlicher Entfernung bereitstellt, die gleiche wie die Aufnahmerichtung des ersten integrierten Kameramoduls des tragbaren Computers ist.

2. Hilfsvorrichtung nach Anspruch 1, ferner umfassend:
ein erstes Blitzmodul, um einen ersten Blitz bereitzustellen, wenn das erste integrierte Kameramodul ein Bild aufnimmt.

3. Hilfsvorrichtung nach Anspruch 1, ferner umfassend:
ein erstes integriertes Blitzmodul, um einen ersten Blitz bereitzustellen, wenn das erste integrierte Kameramodul ein Bild aufnimmt.

4. Hilfsmodul nach Anspruch 1, ferner umfassend:
ein Strahlengang-Änderungsmodul, um die Richtung, die den Brennpunkt in unendlicher Entfernung des optischen Fernsichtmoduls und die Aufnahmerichtung des ersten integrierten Kameramoduls bereitstellt, in derselben senkrechten Fläche anzuordnen.

5. Hilfsvorrichtung nach Anspruch 1, ferner umfassend:
ein Kameramodul zum Aufnehmen eines Bildes der Messperson, die auf eine Bildschirmausgabe blickt, die von einem Bildschirmmodul des tragbaren Computers ausgegeben wird.

6. Hilfsmodul nach Anspruch 5, ferner umfassend:
ein zweites integriertes Blitzmodul, um einen zweiten Blitz bereitzustellen, wenn das Kameramodul ein Bild aufnimmt.

7. Hilfsvorrichtung nach Anspruch 1, wobei ein drahtgebundenes Endgerät des optischen Fernsichtmoduls an einen Klinkenstecker des tragbaren Computers angeschlossen ist.

## Revendications

1. Dispositif auxiliaire pour mesurer des paramètres pour fabriquer des verres de lunettes, qui doit être installé sur un ordinateur portable ayant un premier module d'appareil de prise de vues incorporé, le dispositif auxiliaire comprenant :
un module optique à distance infinie comprenant une source de lumière cible et un verre à foyer infini agencé devant la source de lumière cible pour fournir un foyer de distance infinie aux deux yeux d'un sujet de mesure à partir de la source de lumière cible à travers le verre à foyer infini ; et
- ledit module optique à distance infinie doit être monté de façon amovible sur l'ordinateur portable d'une telle façon que la direction fournissant le foyer à distance infinie est la même que la direction de capture du premier module d'appareil de prise de vues incorporé de l'ordinateur portable.

2. Dispositif auxiliaire selon la revendication 1, comprenant en outre :
un premier module de flash pour fournir un premier flash quand le premier module d'appareil de prise de vues incorporé prend une image.

3. Dispositif auxiliaire selon la revendication 1, comprenant en outre :
un premier module de flash incorporé pour fournir un premier flash lorsque le premier module d'appareil de prise de vues incorporé prend une image.

4. Dispositif auxiliaire selon la revendication 1, comprenant en outre :
un module de changement de chemin optique pour agencer la direction fournissant le foyer à distance infinie du module optique à foyer infini et la direction de capture du premier module d'appareil de prise de vues incorporé dans la même surface verticale.

5. Dispositif auxiliaire selon la revendication 1, comprenant en outre :
un module d'appareil de prise de vues pour prendre une image du sujet de mesure qui regarde une sortie d'écran provenant d'un module d'écran de l'ordinateur portable.

6. Dispositif auxiliaire selon la revendication 5, comprenant en outre :
un second module de flash incorporé pour fournir un second flash quand le module d'appareil de prise de vues prend une image.

7. Dispositif auxiliaire selon la revendication 1, dans lequel un embout de câble du module optique à foyer infini est relié à un port de jack audio de l'ordinateur portable.
